# EUROPÄISCHE PATENTSCHRIFT

(11) **EP 1 005 854 B1**
(45) Veröffentlichungstag und Bekanntmachung des Hinweises auf die Patenterteilung: **03.07.2002**
(21) Anmeldenummer: 99123141.6
(22) Anmeldetag: 19.11.1999
(51) Int. Cl.: A61K 7/32

(54) **Verwendung von Sulfobetainen als Antitranspirantien**
Use of sulfobetaines as antiperspirant
Utilisation de sulfobetaines comme agent antitranspirant

(30) Priorität: 04.12.1998 DE 19855936
(43) Veröffentlichungstag der Anmeldung: 07.06.2000
(73) Patentinhaber: Beiersdorf Aktiengesellschaft, 20245 Hamburg (DE)
(72) Erfinder: Wolf, Florian, Dr., 20251 Hamburg (DE); Keskin, Maike, Dr., 22765 Hamburg (DE)

(56) Entgegenhaltungen:
- DE-A- 2 747 355
- FR-A- 1 067 695
- US-A- 4 183 952
- US-A- 5 676 936

## Beschreibung

Die vorliegende Erfindung betrifft die Verwendung an sich bekannter Substanzen als antitranspirierend wirksame Agentien. Insbesondere betrifft die Erfindung die Verwendung dieser Stoffe in kosmetischen oder dermatologischen Zubereitungen.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Sollen kosmetische oder pharmazeutische Stifte bestimmte Wirkstoffe enthalten, ist denkbar, daß die übrigen Bestandteile mit den Wirkstoffen nicht kompatibel sind. Dies ist besonders häufig der Fall, wenn die Verwendung der kosmetischen Stifte als Deo-Stifte vorgesehen ist, und insbesondere als antitranspirierend wirksame Stifte. Letztere enthalten in der Regel Aluminiumchlorhydrat, welches als kräftige Lewis-Säure gerade für viele Stiftformulierungen nicht verwendbar war. Gerade desodorierende Stifte werden nämlich in der Regel aus Seifen-Glykol-Gelen gebildet, die in dem Umstande begründet liegen, daß niedere Glycole und Glycerin in Gegenwart von Natriumstearat klare, transparente Gele bilden können, welche zusätzlich Alkohol und Wasser aufnehmen können. Solche Formulierungen aber sind nicht mit Aluminiumchlorhydrat verträglich.

Aus Gründen der Verträglichkeit ist es stets zu bevorzugen, selbst bei Verwendung an sich unbedenklicher Substanzen, entsprechende Einsatzkonzentrationen solcher Wirkstoffe möglichst niedrig zu halten.

Eine Aufgabe der vorliegenden Erfindung war es also, Zubereitungen zu entwickeln, welche als Grundlage für kosmetische Desodorantien bzw. Antitranspirantien geeignet sind, und die Nachteile des Standes der Technik nicht aufweisen. Weiterhin war es also eine Aufgabe der Erfindung, kosmetische Grundlagen für kosmetische Desodorantien zu entwickeln, die sich durch gute Hautverträglichkeit auszeichnen.

Bekannt und gebräuchlich sind neben den flüssigen Desodorantien auch feste Zubereitungen, beispielsweise Deo-Stifte ("Sticks"), Puder, Pudersprays, Intimreinigungsmittel usw.

Die US-PS 4,719,103 beschreibt einen Antitranspirantstift auf der Basis einer W/O-Emulsion, welcher einen hohen Wasseranteil enthalte, welcher sich auszeichnet durch einen Gehalt an flüchtigen Silikonkomponenten, ein festes Alkanol sowie Polyglycerinfettsäureester, beispielsweise Polyglycerylisostearat, als Emulgator. Die US-PS 4,704,271 und die US-PS 4,725,431 beschreiben ähnliche Zubereitungen.

Die GB-OS 2 162 439 beschreibt paraffinhaltige Stifte, welche einen hohen Wasseranteil enthalten sollen, wobei die Emulgatoren aus der Gruppe der Metallsalze gewählt werden.

Es war nach all diesem überraschend und nicht vorhersehbar, daß die Verwendung von mono- di- und/oder trimethylammonio-substituierten Sulfonsäuren der allgemeinen Strukturformel 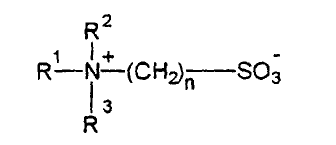 welche im Rahmen der vorliegenden Offenbarung kollektiv als "Sulfobetaïne" bezeichnet werden, als antitranspirierend wirksame Agentien die Nachteile des Standes der Technik beseitigt.

R₁, R₂ und R₃ stellen unabhängig voneinander Wasserstoffatome oder Methylgruppen dar, n nimmt Werte von 1 bis 18 an.

Unter die Sulfobetaine zu rechnen ist insbesondere das Taurin (2-Aminoethansulfonsäure) und dessen Derivate, welches sich durch die Strukturformel 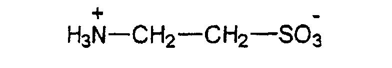 auszeichnet. Im Organismus entsteht es aus Cystein, in der Galle ist es an Cholsäure als Amid gebunden (Taurocholsäure). Taurin kommt in fast allen Säugetierarten vor, spielt eine wichtige Rolle bei der Entwicklung des zentralen Nervensystems und beeinflußt Transportvorgänge 2-wertiger Metall-Ionen, z. B. als Calcium-, Magnesium- u. Zink-Modulator. Die Bedeutung der Taurinzufuhr mit der Nahrung wird in zahlreichen Übersichtsarbeiten diskutiert, wahrscheinlich ist Taurin für einige Säugetiere essentiell, besonders im heranwachsenden Organismus.

Entsprechend der erfindungsgemäßen Verwendung sind die Zubereitungen besonders vorteilhaft dadurch gekennzeichnet, daß die kosmetisch oder pharmazeutisch unbedenklichen Sulfobetaïne in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

Stellen die erfindungsgemäß erhaltenen Zubereitungen Desodorantien/Antitranspirantien dar, so können zusätzlich zu den erfindungsgemäß verwendeten Sulfobetaïnen alle gängigen Wirkstoffe vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, llit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäß erhaltenen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-42 29 737, DE-42 37 081, DE-43 09 372, DE-43 24 219 beschriebenen wirksamen Agenzien.

Weitere übliche Antitranspiranswirkstoffe können ebenfalls vorteilhaft in den erfindungsgemäßen antitranspirierend wirksamen Zubereitungen verwendet werden, insbesondere Adstringentien, beispielsweise basische Aluminiumchloride.

Entsprechend der erfindungsgemäßen Verwendung können die kosmetischen Zubereitungen als Desodorantien bzw. Antitranspirantien bezeichnet werden. Vorteilhaft können sie beispielsweise in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, als Deo-Stifte (Deo-Sticks) und in Form von aus normalen Flaschen und Behältern auftragbaren W/O- oder O/W-Emulsionen, z.B. Crèmes oder Lotionen. Weiterhin können die kosmetischen Desodorantien vorteilhaft in Form von desodorierenden bzw. antitranspirierend wirkenden Tinkturen, desodorierenden bzw. antitranspirierend wirkenden Intimreinigungsmitteln, desodorierenden bzw. antitranspirierend wirkenden Pudern oder desodorierenden bzw. antitranspirierend wirkenden Pudersprays vorliegen.

Als übliche kosmetische Trägerstoffe zur Herstellung der desodorierenden bzw. antitranspirierend wirkendenZubereitungen gemäß der erfindungsgemäßen Verwendung können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten undloder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Emulsionen entsprechend der vorliegenden Erfindungen enthalten einen oder mehrere Emulgatoren, insbesondere vorteilhaft gewählt aus der Gruppe Glycerylstearat im Gemisch mit Ceteareth-20; Sorbitanstearat; Sorbitanoleat; Ceteareth-25; Ceteareth-6 im Gemisch mit Stearylalcohol; Cetylstearylalcohol im Gemisch mit PEG-40-Ricinusöl und Natriumcetylstearylsulfat; Triceteareth-4 Phosphat; Glycerylstearat; Natriumcetylstearylsulfat; Lecithin; Trilaureth-4 Phosphat; Laureth-4 Phosphat; Stearinsäure; Propylenglycolstearat SE; PEG-25-hydriertes Ricinusöl; PEG-54-hydriertes Ricinusöl; PEG-6 Caprylsäure/Caprinsäureglyceride; Sorbitanstearat; Glyceryloleat im Gemisch mit Propylenglycol; PEG-9-Stearat; Glyceryllanolat; Ceteth-2; Ceteth-20; Polysorbat 60; Lanolin; Glycerylstearat im Gemisch mit PEG-100 Stearat; Glycerylmyristat; mikrokristallines Wachs (Cera microcristallina) im Gemisch mit Paraffinöl (Paraffinum liquidum), Ozokerit, hydriertem Ricinusöl, Glyceryl Isostearat und Polyglyceryl-3-Oleat; Glyceryllaurat; PEG-40-Sorbitanperoleat; Laureth-4; Ceteareth-3; Wollwachssäuregemische; Isostearylglycerylether; Cetylstearylalkohol im Gemisch mit Natrium Cetylstearylsulfat; Wollwachsalkoholgemische; Laureth-23; Steareth-2; Glycerylstearat im Gemisch mit PEG-30 Stearat; PEG-40-Stearat; Glycol Distearat; PEG-22-Dodecyl Glycol Copolymer; Polyglyceryl-2-PEG-4 Stearat; Pentaerythrithylisostearat; Polyglyceryl-3 Diisostearat; Ceteareth-20; Sorbitan Oleat im Gemisch mit hydriertem Ricinusöl, Bienenwachs (Cera alba) und Stearinsäure; Natriumdihydroxycetylphosphat im Gemisch mit Isopropylhydroxycetylether; Methylglucosesesquistearat; Steareth-10; PEG-20-Stearat; Steareth-2 im Gemisch mit PEG-8 Distearat; Steareth-21; Steareth-20; Isosteareth-20; Methylglucosedioleat; PEG-7-hydriertes Ricinusöl; Sorbitanoleat im Gemisch mit PEG-2-hydriertem Ricinusöl, Ozokerit und hydriertem Ricinusöl; Sorbitanisostearat im Gemisch mit PEG-2-hydriertem Ricinusöl, Ozokerit und hydriertem Ricinusöl; PEG-45/ Dodecylglycol-Copolymer; Methoxy-PEG-22/Dodecylglycol-Copolymer; hydrierte Cocosfettsäureglyceride; Polyglyceryl-4-Isostearat; PEG-40-Sorbitanperoleat; PEG-40-Sorbitanperisostearat; PEG-20-Glycerylstearat; PEG-20-Glycerylstearat; PEG-8-Bienenwachs; Laurylmethiconcopolyol; Cetyldimethiconcopolyol; Polyglyceryl-2-Iaurat; Isostearyldiglycerylsuccinat; Stearamidopropyl-PG-dimoniumchloridphosphat; PEG-7-hydriertes Ricinusöl; Glycerylstearat, Ceteth-20; Triethylcitrat; PEG-20-Methylglucosesesquistearat; Ceteareth-12; Paraffinöl (Paraffinum liquidum); Glycerylstearatcitrat; Cetylphosphat; Sorbitansesquioleat; Acrylat/C₁₀₋₃₀-Alkylacrylat-Crosspolymer; Sorbitanisostearat; Methylglucosesesquistearat; Triceteareth-4-Phosphat; Trilaureth-4-Phosphat; Polyglycerylmethylglucosedistearat; Poloxamer 101; Kaliumcetylphosphat; Isosteareth-10; Polyglyceryl-2-sesquiisostearat; Ceteth-10; Polyglyceryl-2 Dipolyhydroxystearat; Oleth-20; Isoceteth-20; Glycerylisostearat; Polyglyceryl-3-Diisostearat; Glycerylstearat im Gemisch mit Ceteareth-20, Ceteareth-12, Cetylstearylalcohol und Cetylpalmitat; Cetylstearylalcohol im Gemisch mit PEG-20 Stearat; Glycerylstearat; PEG-30-Stearat.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilicat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Zuberereitungen gemäß der vorliegenden Erfindung können sich auch durch einen Gehalt an Tensiden auszeichnen.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische lonen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische lonen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wäßrigem Medium keine lonen.

### A. Anionische Tenside

Vorteilhaft zu verwendende anionische Tenside sind
Acylaminosäuren (und deren Salze), wie
1. Acylglutamäte, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. AcylLactylate, lauroyllactylat, Caproyllactylat
6. Alaninate
Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,
Sulfonsäuren und Salze, wie
1. Acyl-isethionate, z.B. Natrium-/Ammoniumcocoyl-isethionat,
2. Alkylarylsulfonate,
3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie
Schwefelsäureester, wie
1. Alkylethersulfat, beispielsweise Natrium-, Ammonium-, Magnesium-, Ml PA-, TIPA-Laurethsulfat, Natriummyrethsulfat und Natrium C₁₂₋₁₃ Parethsulfat,
2. Alkylsulfate, beispielsweise Natrium-, Ammonium- und TEA- Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats
Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, AlkylSulfobetain, AlkylamidopropylSulfobetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxyliertel propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist femer die Verwendung einer Kombination von anionischen und/oder amphoteren Tensiden mit einem oder mehreren nicht-ionischen Tensiden.

Es folgen vorteilhafte Ausführungsbeispiele der vorliegenden Erfindung.

### Beispiel 1

| O/W-Lotion | |
|---|---|
| | Gew.-% |
| Paraffinöl | 5,00 |
| Isopropylpalmitat | 5,00 |
| Cetylalkohol | 2,00 |
| Bienenwachs | 2,00 |
| Ceteareth-20 | 2,00 |
| PEG-20-Glycerylstearat | 1,50 |
| Glycerin | 3,00 |
| N,N-Dimethyltaurin | 1,00 |
| Parfum, Konservierungsstoffe | q.s. |
| Wasser, VES | ad 100,00 |

### Beispiel 2

| Seifenstück | |
|---|---|
| | Gew.-% |
| Na-Salz aus Talgfettsäuren | 60,00 |
| Na-Salz aus Kokosöl | 28,00 |
| Natriumchlorid | 0,50 |
| N,N-Dimethyltaurin | 1,00 |
| Parfum, Konservierungsstoffe | q.s. |
| Wasser | ad 100,00 |

### Beispiel 3

| Aerosolspray | |
|---|---|
| | Gew.-% |
| Octyldodecanol | 0,50 |
| N,N,N-Trimethylammoniotaurin | 0,20 |
| Parfum, Konservierungsstoffe | q.s. |
| Ethanol | ad 100,00 |

Die durch Zusammenmischung der jeweiligen Bestandteile erhaltene flüssige Phase wird zusammen mit einem Propan-Butan-Gemisch (2:7) im Verhältnis 39:61 in Aerosolbehälter abgefüllt.

## Patentansprüche

1. Verwendung von mono- di- und/oder trimethylammonio-substituierten Sulfonsäuren der allgemeinen Strukturformel 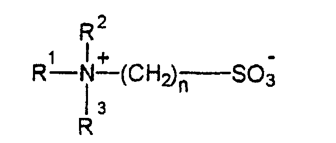 als antitranspirierend wirksame Agentien um die Bildung des Schweißes zu reduzieren, wobei R₁, R₂ und R₃ unabhängig voneinander Wasserstoffatome oder Methylgruppen darstellen und n Werte von 1 bis 18 annimmt.

2. Verwendung nach Anspruch 1, **dadurch gekennzeichnet, daß** als mono- di- und/oder trimethylammonio-substituierten Carbonsäure das N,N-Dimethyltaurin und/oder das N,N,N-Trimethylammoniotaurin eingesetzt wird.

3. Verwendung nach Anspruch 1 in Zubereitungen, die **dadurch gekennzeichnet sind, daß** das oder die kosmetisch oder pharmazeutisch unbedenklichen Sulfobetaïne in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

## Claims

1. Use of mono- di- and/or trimethylammonio-substituted sulphonic acids of the general structural formula 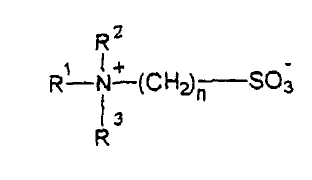 as agents with an antiperspirant action in order to reduce the formation of perspiration, where R₁, R₂ and R₃, independently of one another, are hydrogen atoms or methyl groups, and n assumes values from 1 to 18.

2. Use according to Claim 1, **characterized in that** the mono- di- and/or trimethylammonio-substituted carboxylic acid used is N,N-dimethyltaurine and/or N,N,N-trimethylammoniotaurine.

3. Use according to Claim 1 in preparations which are **characterized in that** the cosmetically or pharmaceutically safe sulphobetaines is or are present in concentrations of 0.01 - 10.00% by weight, preferably 0.05 - 5.00% by weight, particularly preferably 0.1 - 3.00% by weight, in each case based on the total weight of the composition.

## Revendications

1. Utilisation d'acides sulfoniques mono-, di- et/ou triméthylammonio-substitués de formule structurale générale 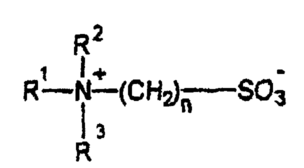 dans laquelle R₁, R₂ et R₃ représentent, indépendamment les uns des autres, des atomes d'hydrogène ou des groupes méthyle, et n prend des valeurs de 1 à 18, en tant qu'agents à action anti-transpirante, en vue de réduire la formation de la sueur.

2. Utilisation selon la revendication 1, **caractérisée en ce que** la N,N-diméthyltaurine et/ou la N,N,N-triméthylammoniotaurine est utilisée en tant d'acide carboxylique mono-, di- et/ou triméthylammonio-substitué.

3. Utilisation selon la revendication 1 dans des préparations qui sont **caractérisées en ce que** la ou les sulfobétaïne(s) sans risque du point de vue cosmétique ou pharmaceutique est (sont) présente(s) en concentrations de 0,01 à 10,00 % en poids, de préférence de 0,05 à 5,00 % en poids, particulièrement préférablement de 0,1 à 3,00 % en poids, dans chaque cas par rapport au poids total de la composition.
